# EUROPEAN PATENT APPLICATION

(11) **EP 4 733 312 A1**
(43) Date of publication of application: **29.04.2026**
(21) Application number: 24824820.5
(22) Date of filing: 18.06.2024
(51) Int. Cl.: C07K 14/315, A61K 39/116, G01N 33/569

(54) **MULTI-EPITOPE ANTIGEN, IMMUNOGENIC COMPOSITION COMPRISING SAID ANTIGEN, PNEUMOCOCCAL DIAGNOSTIC KIT, AND USES OF SAID ANTIGEN**

(30) Priority: 22.06.2023 BR 102023012657
(71) Applicant: INSTITUTO BUTANTAN, 05503900 São Paulo - SP (BR)
(72) Inventor: ALVES, Vítor Dos Santos, 05359-001 São Paulo - SP (BR); GONÇALVES, Viviane Maimoni, 05416-010 São Paulo - SP (BR)
(74) Representative: Isern Patentes y Marcas S.L.
(86) International application number: PCT/BR2024/050260
(87) International publication number: WO 2024/259508

(57) **Abstract**

The present invention relates to a multi-epitope antigen comprising 20 epitope blocks of 20 to 36 amino acids from proteins selected from the group consisting of NanA, PcsB, PhtD, Ply, PncO, StkP, PspA-F1 and PspA-F2. Additionally, the present invention relates to an immunogenic composition comprising said antigen, and a pharmaceutically acceptable vehicle and/or adjuvant. Furthermore, a pneumococcal diagnostic kit comprising said antigen or a biologically active fragment thereof bound to a detectable fraction; an antibody generated by said antigen; and instructions for use is also disclosed. Moreover, the present invention relates to the use of said antigen for the preparation of a vaccine to prevent diseases caused by pneumococcal bacteria, and for the production of monoclonal and polyclonal antibodies. Finally, the present invention relates to an *in vitro* diagnostic method for pneumococcus comprising applying said antigen or a biologically active fragment thereof bound to a detectable fraction, in contact with selected body fluid samples from blood, mucus expelled from the lower airways and urine.

## Description

### Field of Application:

The present invention falls within the field of preparations for medical purposes, more specifically, in the area of medicinal preparations containing antigens or antibodies, since it relates to a pneumococcal vaccine using bioinformatics computational techniques composed of epitopes derived from different pneumococcal proteins.

### Foundations of Invention and State of the Art:

*Streptococcus pneumoniae* or pneumococcus is a Gram-positive, encapsulated bacterium that has 100 immunologically distinct serotypes of worldwide epidemiological importance in the distribution of invasive pneumococcal diseases (bacteremic pneumonia, meningitis, sepsis, and arthritis) and non-invasive diseases (sinusitis, acute otitis media, conjunctivitis, bronchitis, and pneumonia). Pneumococcal disease can lead to serious infections in the lungs (pneumonia), blood (bacteremia - dissemination of bacteria through the blood / sepsis - generalized infection), and the membrane lining the brain (meningitis). Bacteremia and meningitis are invasive pneumococcal infections, usually very serious, leading to hospitalization or even death.

Prevention through vaccination against pneumococcal disease is the best way to protect against pneumococcus. However, pneumococcal vaccines rank among the most expensive for adults on the Center for Disease Control *and* Prevention (CDC) price list https://www.cdc.gov/vaccines/programs/vfc/awardees/vaccine-management/price-list/index.html), making them difficult to access in underdeveloped countries.

An additional aggravating factor is that these vaccines prevent a maximum of 20 serotypes, compared to the 100 existing serotypes. This is because pneumococcal vaccines on the market are based on polysaccharides from the pathogen's capsule, which in turn are highly variable (100 serotypes), and existing vaccines prevent only 20% of this total and are subject to the phenomenon of serotype replacement.

In view of the aforementioned technical problem, the present invention proposes a serotype-independent and cheaper pneumococcal vaccine, whose stability, immunogenicity, and antigenicity properties have been previously evaluated by state-of-the-art computational tools and techniques, ensuring the selection of candidates with the greatest potential, saving time and money in experimental analyses.

Pneumococcal proteins, being more conserved among different serotypes, are very promising for generating serotype-independent vaccines. The sophistication of computational tools has made it possible to screen B and T cell epitopes among these proteins and has enabled the design of antigens based on the union of different epitopes, whose stability and immunological potential can be predicted.

Given this, the antigens proposed by the present invention were obtained by computational techniques. These bioinformatics studies comprise 20 epitopes, derived from 8 different pneumococcal proteins, all involved in the virulence process of pneumococcus, and with defined data on antigenicity, population coverage, and antigen stability.

Some prior art documents describe vaccines that include pneumococcal antigens.

Brazilian patent application no. PI 0813892-3 A2, published on May 9, 2017, in the name of NOVARTIS AG., entitled: "COMPOSIÇÕES QUE COMPREENDEM ANTÍGENOS PNEUMOCÓCICOS" refers to various combinations of whole protein antigens, and also fusion proteins of 2 antigens, but not the use of epitopes of these antigens, as proposed by the present invention. The aforementioned document differs from the present invention because it uses a mixture of several whole proteins or fusion proteins, not a single polypeptide chain composed of a single protein molecule containing the desired epitopes of the 8 different antigens and not found in nature as such. In an innovative way, the present invention comprises combining the 20 epitopes of the 8 antigens into a single polypeptide chain that does not exist in *Streptococcus pneumoniae,* whereas the document proposes the use of whole antigens, which cannot be classified as a multi-epitope vaccine.

International patent application no. PCT/IL2010/001009, published under no. WO 2011/067758 (A2) on June 9, 2011, in the name of PROTEA VACCINE TECHNOLOGIES LTD., entitled *"IMMUNOGENIC FRAGMENTS AND MULTIMERS FROM STREPTOCOCCUS PNEUMONIAE PROTEINS"* explicitly states that the revealed sequences have either 51 to 250 amino acids, or 51 to 100 amino acids. These sequences obviously contain epitopes within them; otherwise, they would be useless as a vaccine. However, they are fragments of the entire antigens, not epitopes of those antigens, since epitopes typically have smaller sizes, as in the case of the present invention. The document further proposes obtaining multimers of the 51 to 250 amino acid fragments of the proteins, which can be obtained recombinantly as fusion proteins with a maximum of 1000 amino acids. Therefore, the fusions proposed in this document differ significantly from the proposal of the present invention, which uses only the amino acid sequences corresponding to the epitopes. Thus, the largest epitope selected for the present invention has only 36 amino acids, compared to more than 51 amino acids for the aforementioned international application. Furthermore, the epitopes of the present invention are linked by much smaller and distinct spacers than those proposed in this document. In conclusion, the proposal in this document cannot be classified as referring to multi-epitopes, since it uses the term multimer, not multi-epitope, employs antigens distinct from those of the present invention (wall and membrane antigens), and also uses a different spacer.

International patent application No. PCT/II,2010/000439, published under No. WO 2010/150242 (A2) on December 29, 2010, in the name of PROTEA VACCINE TECHNOLOGIES LTD., entitled *"IMMUNOGENIC STREPTOCOCCUS PNEUMONIAE PEPTIDES AND PEPTIDE-MULTIMERS"* employs fragments of cell wall and membrane proteins of *Streptococcus pneumoniae* but uses fragments of 9 to 50 amino acids. Furthermore, unlike the present invention, the aforementioned document employs pneumolysin or *heat-shock protein* 60 (HSP60) as a carrier protein for the selected antigen fragments, while the present invention proposes 3 arrangements of the epitope sequence according to the physicochemical characteristics analyzed *in silico* to generate stable molecules, without the need to add a carrier protein. This difference is extremely relevant both from the point of view of the immune response, since the carrier protein excels in stimulating the immune response, and from the point of view of the acquisition process, which is more complex in the case of binding the fragments to the carrier protein.

U.S. Patent Application No. 2015/0079132 (A1), published March 19, 2015, in the name of PATH, entitled "EVOKING PROTECTION AGAINST *STREPTOCOCCUS PNEUMONIAE* INCORPORATING B-CELL AND T-CELL PNEUMOCOCCAL PROTEIN ANTIGENS AND PNEUMOCOCCAL POLYSACCHARIDES DELIVERED CONCOMITANTLY" refers to protein fragments containing B-cell epitopes (which stimulate antibody production) of only 3 proteins: HSP70, PspA, and pneumolysin, and a larger number of proteins or fragments thereof that stimulate T cells. The aforementioned document proposes using mixtures and/or fusions of antigens from these two groups, with the fusions being of 2 or 3 protein fragments, not epitopes. On the other hand, the proposal of the present invention identified in the 8 antigens the epitopes that stimulate both B and T cells. Furthermore, only some sequences disclosed in that US patent application are actually considered epitopes; all other polypeptide sequences shown are considerably larger protein fragments. Finally, the ligands employed are much larger than those employed in the present invention and only serve to separate the fragments from the antigens, while the ligands of the present invention serve to stimulate the recognition and processing of the molecule by the immune system.

Chinese patent application no. CN 105131093 (A), published on December 9, 2015, in the name of CHANGCHUN BCHT BIOTECHNOLOGY CO and JILIN UNIVERSITY, entitled *"STREPTOCOCCUS PNEUMONIA PROTEIN ANTIGEN, PREPARATION, THEREFOR AND APPLICATIONS"* deals with obtaining a pneumolysin protein mutant with 2-point mutations with the substitution of cysteine for glycine at position 428 and of tryptophan for phenylalanine at position 433, called PlyM2. It compares this double mutant with mutants containing only one of the alterations and shows an improvement in the immune response when other proteins (PsaA, PcsB, StkP, PspA, PhtD and PcpA) were co-administered, that is, only mixed, with PlyM2. Although the present invention contains epitopes of some of these proteins (pneumolysin, PcsB, PhtD, StkP, PspA-F1 and PspA-F2, in addition to two that are not in the Chinese application, NanA and PncO), there is little relation between this document and the present invention, since the proposal of the present invention is to use only the epitopes of proteins and genetically fuse them into a single polypeptide chain. On the other hand, the aforementioned Chinese document does not even mention protein fusion, limiting itself to proposing co-administration, while the present invention is based on the most advanced immunobioinformatics techniques.

The article in the name of Mamede, LD et al., entitled: "REVERSE AND STRUCTURAL VACCINOLOGY APPROACH TO DESIGN A HIGHLY IMMUNOGENIC MULTI-EPITOPE SUBUNIT VACCINE AGAINST STREPTOCOCCUS PNEUMONIAE INFECTION", published in Infection, Genetics and Evolution 85 (2020) 104473, available online on July 24, 2020, differs from the present invention in several ways: (i) in that article, the selection of T and B lymphocyte epitopes was based on only one and two predictors, respectively, while the present invention used four predictors for B lymphocytes (*BepiPred, SVMtrip, ABCPred, and BCPred*) and five for T lymphocytes (*NetMHCII, NetMHCIIpan, NetCTLpan, NetMHC, and NetTepi);* (ii) the aforementioned article did not predict the allergenicity of the selected epitopes, unlike the present invention which used two predictors for this purpose *(AllergenFP* and *Aller TOP*); (iii) In the present article, the two constructs are proposed based on epitope order: in the first, the order was random, and in the second, which was eventually abandoned, the Protparam hydropathic index was used, without going into detail about the logic employed. In contrast, the present invention introduced the concept of blocks, which were randomly arranged in the construction of the sequences. Furthermore, this concept was used to order the epitopes in such a way as to place the most hydrophilic ones at the ends of the protein and the most hydrophobic ones in the center of the polypeptide chain. Additionally, it was identified that epitopes containing the amino acid valine in the N-terminal position can make the protein more stable, a fact unknown in the prior art in this technical field and observed through the combination of appropriate bioinformatics tools. (iv) Finally, there are also differences regarding the generation and analysis of the 3D models, where in the present invention the models were generated using AlphaFold2 and validated with Prosa-Web, PROCHECK from SAVES, and QMEAN, programs not used in this cited article.

The article in the name of Shafaghi, M et al., entitled: "IMMUNOINFORMATICS - AIDED DESIGN OF A NEW MULTI - EPITOPE VACCINE ADJUVANTED WITH DOMAIN 4 OF PNEUMOLYSIN AGAINST STREPTOCOCCUS PNEUMONIAE STRAINS" , published in BMC Bioinformatics (2023) 24:67, available online on February 24, 2023, uses only two proteins (PhtD and PspA) to generate the epitopes, and an 111-amino acid domain of pneumolysin (Ply) as an adjuvant in the vaccine. In contrast, the present invention utilizes epitopes shorter than 37 amino acids obtained from 8 different proteins. The immunological profile explored also differs, since the present invention uses B, MHC I, and MHC II epitopes, employing four predictors for B lymphocytes (*BepiPred, SVMtrip, ABCPred* and *BCPred*) and five for T lymphocytes (*NetMHCII, NetMHCIIpan, NetCTLpan, NetMHC* and *NetTepi),* while the aforementioned article uses only B and MHC II epitopes, the latter based on only two predictors (*NetMHCIIpan* and *IEDB*)*.*

Thus, it is important to emphasize that any proposed pneumococcal vaccine, independent of serotypes, will make use of the bacterium's protein antigens, since it is precisely these antigens that will give the desired characteristics to the new vaccine. The novelty, not only of the present multi-epitope vaccine proposal, but of all the documents cited in the prior art, lies in the choice and combination of antigens, since it would be technically and economically unfeasible to produce all the protein antigens of the microorganism to compose a vaccine.

Thus, no person skilled in the art, employing or not bioinformatics tools, could achieve the same combination of epitopes arranged in 3 different orderings in a single polypeptide chain only by logical analysis and inference based on the prior art cited.

Therefore, although vaccine development using immunobioinformatics tools already exists, no prior art document discloses the vaccine of the present invention composed of 20 epitopes, derived from 8 different pneumococcal proteins, all involved in the virulence process of pneumococcus and with defined antigenicity, population coverage and stability data.

### Invention Summary:

As already mentioned, the present invention will provide significant advantages for the development of an alternative multi-epitope pneumococcal vaccine.

In a first aspect, the present invention relates to a multi-epitope antigen comprising 20 epitope blocks of 20 to 36 amino acids from proteins selected from the group consisting of NanA, PcsB, PhtD, Ply, PncO, StkP, PspA-F1 and PspA-F2.

In a second aspect, the present invention relates to an immunogenic composition comprising said antigen, and a pharmaceutically acceptable vehicle and/or adjuvant.

In a third aspect, the present invention relates to an early pneumococcal diagnostic kit comprising said multi-epitope antigen or a biologically active fragment thereof bound to a detectable fraction; at least one antibody generated by said multi-epitope antigen; and instructions for use.

In a fourth aspect, the present invention relates to the use of said antigen in the preparation of a vaccine to prevent diseases caused by pneumococcal bacteria.

In a fifth aspect, the present invention relates to the use of said antigen for the production of monoclonal and polyclonal antibodies.

In a sixth aspect, the present invention relates to an *in vitro* method for early diagnosis of pneumococcus comprising applying said multi-epitope antigen or a biologically active fragment thereof bound to a detectable fraction, in contact with samples of selected body fluids from blood, mucus expelled from the lower airways and urine.

### Brief description of the figures:

The present invention, together with its additional advantages, can be better understood by reference to the attached images and the following description.
Figure 1 graphically presents the epitope coverage in the world population.
Figure 2 presents the 3D models of the SEQ ID NO:21 to 23 antigens.
Figures 3 A-C show the validation of the 3D models of the SEQ ID NO: 21 to 23 antigens, which was performed using different bioinformatics programs (QMEAN, Prosa-Web and Procheck from SAVES).

### Detailed description of the invention:

Although the present invention may be susceptible to different embodiments, a preferred embodiment is shown in the following detailed discussion, with the understanding that this embodiment should be considered an exemplification of the principles of the invention and it is not intended to limit the present invention to what has been described in this report.

The present invention relates to a multi-epitope antigen comprising blocks of 20 to 36 amino acid epitopes from proteins selected from the group consisting of NanA, PcsB, PhtD, Ply, PncO, StkP, PspA-F1 (family 1 PspA) and PspA-F2 (family 2 PspA).

Preferably, said epitopes are selected from the group consisting of MHC I, MHC II and B epitopes.

Preferably, the multi-epitope antigen of the present invention comprises 20 blocks of 20 to 36 amino acid epitopes from said proteins.

More preferably, the antigen of the present invention comprises 3 blocks of the NanA protein, 2 blocks of the PcsB protein, 3 blocks of the PhtD protein, 2 blocks of the Ply protein, 3 blocks of the PncO protein, 3 blocks of the StkP protein, 2 blocks of the PspA-F1 protein, and 2 blocks of the PspA-F2 protein.

More preferably, said epitope blocks are selected from the group consisting of amino acid sequences as set forth by SEQ ID NOs: 1 to 20, wherein:
(i) SEQ ID NOs: 1 to 3 refer to the NanA protein blocks;
(ii) SEQ ID NOs: 4 and 5 refer to the PcsB protein blocks;
(iii) SEQ ID NOs: 6 to 8 refer to the PhtD protein blocks;
(iv) SEQ ID NOs: 9 and 10 refer to the Ply protein blocks;
(v) SEQ IDs NOs: 11 to 13 refer to the PncO protein blocks;
(vi) SEQ IDs NOs: 14 to 16 refer to the StkP protein blocks;
(vii) SEQ IDs NOs: 17 and 18 refer to the PspA-F1 protein blocks
(viii) and SEQ IDs NOs: 19 and 20 refer to the PspA-F2 protein blocks.

**Table 1 - Epitope blocks of NanA, PcsB, PhtD, Ply, PncO, StkP, PspA-F1 and PspA-F2 proteins:**

| SEQ ID NO: | Protein | Epitope | GRAVY |
|---|---|---|---|
| 1 | NanA | TPDGKATDYRVVVDPVKPAY | -0,580 |
| 2 | | LENATVHMEFKPDAKAPAFYNLFS | -0,217 |
| 3 | | MAVYNNTATLEGRGSDGKQF | -0,715 |
| 4 | PcsB | MSEIVSANNKMLEQQKADKK | -1,090 |
| 5 | | DNKISNLTAQQQEAQKQVDQIQEQ | -1,571 |
| 6 | PhtD | AGKYTTEDGYIFDPRDITSDEGDAY | -1,084 |
| 7 | | KVGDGYVFEENGVPRYIPAK | -0,580 |
| 8 | | GKYYVYLKDAAHADNIRTKE | -1,025 |
| 9 | Ply | VNFKQIYYTVSVDAVKNPGDVFQDTVTVED LKQRGI | -0,264 |
| 10 | | QVYLKLETTSKSDEVEAAFE | -0,535 |
| 11 | PncO | GLDLLLPSILFALPHFSSLPSLLDIFI | 1,456 |
| 12 | | LIIQNYWQFSSQIGNFVWIQ | 0,275 |
| 13 | | YSGTNFAELGIYITLFFLTP | 0,785 |
| 14 | StkP | EKVEEGRIIRTDPGAGTGRK | -1,245 |
| 15 | | VDLNKTRVKISIYKPKTTSATP | -0,591 |
| 16 | | VTTAPAGSVEGMVVEQSPRA | 0,105 |
| 17 | PspA-F1 | AAQRKYDEDQKKTEEKAKET | -2,465 |
| 18 | | ARRSEEEYNRLPQQQLPKAE | -1,975 |
| 19 | PspA-F2 | KKAYEEAKKKAEDAQKKYDEGQKKTEEKA RK | -2,487 |
| 20 | | EVQNAYVKYQRVQRNSRLNEKER | -1,848 |

Additionally, the antigen of the present invention comprises the ligands "GPGPG" (SEQ ID NO:24) and "KK", wherein the ligand of SEQ ID NO: 24 is used to separate the blocks, and the "KK" is used to separate each epitope within the blocks.

More specifically, "KK" is a ligand that is a target sequence of cathepsin B and enhances the presentation of MHC-II antigens, the "GPGPG" (SEQ ID NO:24) is a ligand used to induce an HTL response (CD4+ lymphocytes).

Additionally, the antigen of the present invention comprises a methionine (M) at the beginning of the amino acid sequence thereof formed by the 20 epitopes, which may or may not be part of said epitopes.

In one embodiment of the present invention, the antigen comprises a random order of said 20 epitopes linked by the ligands "GPGPG" (SEQ ID NO: 24) and "KK".

In a preferred embodiment, the antigen comprises the following epitope order: SEQ ID NO: 16- SEQ ID NO: 15- SEQ ID NO: 14- SEQ ID NO: 5- SEQ ID NO: 4- SEQ ID NO: 9- SEQ ID NO: 10- SEQ ID NO: 2- SEQ ID NO: 3- SEQ ID NO: 1- SEQ ID NO: 8- SEQ ID NO: 7- SEQ ID NO: 6- SEQ ID NO: 12- 13- SEQ ID NO: 11- SEQ ID NO: 17-SEQ ID NO: 18- SEQ ID NO: 19 and - SEQ ID NO: 20.

In a preferred embodiment, the antigen comprises the following epitope order: SEQ ID NO:15- SEQ ID NO:20- SEQ ID NO:14- SEQ ID NO:6- SEQ ID NO:3- SEQ ID NO:17- SEQ ID NO:7- SEQ ID NO:10- SEQ ID NO:9- SEQ ID NO:13- 11- SEQ ID NO:12- SEQ ID NO:16- SEQ ID NO:2- SEQ ID NO:1- SEQ ID NO:8- SEQ ID NO:4-SEQ ID NO:5- SEQ ID NO:18 and - SEQ ID NO:19.

In a preferred embodiment, the antigen comprises the following epitope order: SEQ ID NO: 3- SEQ ID NO:20- SEQ ID NO:14- SEQ ID NO:6- SEQ ID NO:17- SEQ ID NO:15- SEQ ID NO:7- SEQ ID NO:10- SEQ ID NO:9- SEQ ID NO:13- SEQ ID NO:11-SEQ ID NO:12- SEQ ID NO:16- SEQ ID NO:2- SEQ ID NO:1- SEQ ID NO:8- SEQ ID NO:4- SEQ ID NO:5- SEQ ID NO:18- and SEQ ID NO:19.

In a preferred embodiment, the antigen of the present invention consists of the amino acid sequence as set forth by SEQ ID NOS: 21 to 23.

Although not claimed, the process of constructing said antigens is fundamental to obtaining the essential characteristics of the antigens of the present invention.

The rational design of a vaccine using computational techniques, which in biology are called bioinformatics, and the consolidated knowledge of the state of the art on the pathogen (pneumococcus), as well as the immunology of vaccines, allows the creation of safer and more immunogenic vaccines.

Vaccines are safe because, using such tools, it is possible to select, from among the various proteins of interest, the smallest units recognizable by the immune system, called epitopes, capable of generating a response that stimulates the major arms of immunity - humoral (antibodies) and cellular (CD4+ and CD8+ lymphocytes). Using epitopes, it is possible to discard the parts of the proteins that would not serve to mount the immune response and, at the same time, are a threat to vaccine safety, as they increase the chances of allergies and autoimmunity.

More immunogenic due to the rational selection of epitopes. For the present invention, epitopes were selected that stimulate the humoral and cellular arms simultaneously, versatile epitopes derived from important pneumococcal proteins that are not allergenic or present autoimmunity in humans.

To this end, the following bioinformatics steps were performed:

First, a polypeptide chain of antigen epitopes with immunological potential against *Streptococcus pneumoniae* was constructed. The construction process begins with the selection of proteins related to the pathogen in the literature, and obtaining their sequences from GenBank. The sequences are then subjected to B-cell epitope screening, using the BepiPred, SVMtrip, ABCPred and BCPred programs, while the outputs are stored in an Excel spreadsheet.

In parallel, analyses regarding MHC II epitopes were performed using the NetMHCII and NetMHCIIpa predictors, and analyses using the NetCTLpan, NetMHC, and NetTepi predictors for predicting MHC I epitopes were also conducted. All human alleles were included in the analyses for the MHC-I and MHC-II predictors. The predicted epitopes were then subjected to cluster analysis in Epitope Cluster Analysis (IEDB) *(http:*//*tools.iedb.org*/*cluster*/*)* to identify overlaps and select epitopes with potent immunological responses.

After obtaining potent epitopes for all eight proteins used, antigenic properties were analyzed using the Vaxijen program, while allergenicity was verified using AllergenFP and AllerTOP. Those epitopes that were not antigenic and/or allergenic by either of the two predictors were eliminated.

Finally, population coverage was estimated using the IEDB Population Coverage tool (http://tools.iedb.ore/population/).

Once the most potent, immunogenic, and non-allergenic epitopes were obtained, and the linkers ("KK" and "GPGPG" of SEQ ID NO: 24) were selected, the epitope sequences were organized according to two logics: blocks or *Grand average of hydropathicity* (GRAVY) index of each epitope.

In a preferred order, the epitopes of each protein were called blocks, totaling 8 blocks. Thus, the PLY block would correspond to the epitopes VNFKQIYYTVSVDAVKNPGDVFQDTVTVEDLKQRGI (SEQ ID NO: 9) and QVYLKLETTSKSDEVEAAFE (SEQ ID NO: 10), for example. These blocks were named from 1 to 8 and a random order was made in Excel, defining the order of the present sequence (SEQ ID NO: 21). The ligand "GPGPG" (SEQ ID NO: 24) was used to separate the blocks, while the ligand "KK" was used to separate each epitope within the blocks.

A second preferred order was based on the physicochemical properties of the epitopes predicted by Expasy Protparam.

It is worth noting that Expasy Protparam (https://web.expasy.org/protparam/), a program for predicting amino acid characteristics, was used to predict the GRAVY index, which measures hydrophobicity for each epitope. Epitopes with more negative GRAVY values are more hydrophilic than those with more positive values. Having the GRAVY value for each of the epitopes (Table 1), the epitopes were ordered so that the most hydrophobic ones were in the center of the sequence and the most hydrophilic ones at the ends. In this logic, the concept of blocks was not used.

Surprisingly, it was identified that the stability of the proteins increased when an epitope with the amino acid valine (V) was present in the N-terminal region of the sequence (beginning). Based on this finding, the epitope AAQRKYDEDQKKTEEKAKET (SEQ ID NO:17), which is quite hydrophilic, was replaced by the epitope VDLNKTRVKISIYKPKTTSATP (SEQ ID NO:15), swapping their positions (SEQ ID NO: 22).

In a third preferred sequence derived from the selected epitopes and the linkers that space them the sequence organized based on GRAVY was conserved, modifying only the epitope in the N-terminal region of the protein sequence, in this case the epitope AAQRKYDEDQKKTEEKAKET (SEQ ID NO: 17) changed position with the epitope MAVYNNTATLEGRGSDGKQF (SEQ ID NO: 3) thus forming the third preferred multiepitope antigen sequence (SEQ ID NO: 23).

After constructing the amino acid sequences that form the multi-epitope antigens, allergenicity and immunogenicity were reassessed.

The prediction of the antigen's three-dimensional (3D) structure was performed using the AlphaFold2 program *(https:*//*colab.research.google. com*/*github*/*sokrypton* /*ColabFold*/*blob*/*main*/*AlphaFold2.ipy nb#scrollTo=KK7X9T44pWb7*)*.*

Subsequently, refinement was performed using GalaxyWeb *(https:*//*galaxy.seoklab.org*/*cgi-bin*/*submit.cgi?type=REFINE),* and validation was performed using SAVES PROCHECK (*https:*/*saves.mbi.ucla.edu*/)*,* Prosa-Web *(https:*//*prosa.services.came.sbg.ac.at*/*prosa.php*) and QMEAN (*https:*//*swissmodel.expasy.org*/*qmean*/.)

Thus, as mentioned above, the antigens of the present invention were constructed using both block logic and the GRAVY index of each epitope, wherein in a preferred embodiment of the present invention the antigens consist of the amino acid sequence as set forth by SEQ ID NO: 21 (block logic), SEQ ID NO: 22 (GRAVY logic) and SEQ ID NO: 23 (GRAVY logic).

Additionally, the present invention relates to an immunogenic composition comprising the antigen of the present invention, and a pharmaceutically acceptable vehicle and/or adjuvant.

Preferably, said immunogenic composition is a pneumococcal vaccine.

A pharmaceutically acceptable vehicle is understood to be a non-toxic, inert solid, semi-solid liquid excipient, diluent, auxiliary formulation of any kind, or simply a sterile aqueous medium, such as saline solution. Some examples of materials that can serve as pharmaceutically acceptable vehicles are sugars, such as lactose, trehalose, glucose, and sucrose; starches, such as corn starch and potato starch, cellulose and its derivatives, such as sodium carboxymethylcellulose, ethylcellulose and cellulose acetate, cyclodextrin; oils, such as peanut oil, cottonseed oil, sunflower oil, sesame oil, olive oil, corn oil and soybean oil; glycols, such as propylene glycol, polyols, such as glyceryl glycol, sorbitol, mannitol, and polyethylene; Esters, such as ethyl laurate, ethyl oleate, agar; polymers, such as poly(glycerol-adipate-co-ω-pentadecalactone) (PGA-co_PDL), poly(lactic-co-glycolic acid) (PLGA) and chitosan; buffering agents, such as aluminum hydroxide and magnesium hydroxide; alginic acid; pyrogen-free water; isotonic saline, Ringer's solution; ethyl alcohol and phosphate buffer solutions, as well as other compatible non-toxic substances used in pharmaceutical formulations.

A pharmaceutically acceptable adjuvant is any adjuvant known in the state of the art to be pharmaceutically acceptable for obtaining a vaccine.

In one embodiment, said immunogenic composition is administered parenterally, which includes subcutaneous, intravenous, epidural, intramuscular, delivery pumps, or infusion.

In another embodiment, said immunogenic composition may be administered nasally, directed or not to the lungs.

Additionally, the present invention relates to a pneumococcal diagnostic kit comprising the antigen of the present invention or a biologically active fragment thereof linked to a detectable fraction; or the antigen of the present invention and antibodies generated therefrom; and instructions for use.

Preferably, said kit is an immunochromatographic kit or an ELISA (enzyme-linked immunosorbent assay).

Additionally, the present invention relates to the use of the antigen of the present invention for the preparation of a vaccine to prevent diseases caused by the pneumococcal bacterium.

The aforementioned diseases are selected from the group consisting of invasive pneumococcal diseases (bacteremic pneumonia, meningitis, sepsis, and arthritis) and non-invasive diseases (sinusitis, acute otitis media, conjunctivitis, bronchitis, and pneumonia).

Additionally, the present invention relates to the use of the antigen of the present invention for the production of monoclonal and polyclonal antibodies.

Moreover, the present invention relates to an *in vitro* method for early diagnosis of pneumococcus comprising applying the antigen of the present invention or a biologically active fragment thereof bound to a detectable fraction in contact with samples of body fluids such as blood, mucus expelled from the lower airways and urine.

Additionally, the present invention relates to a method for preventing diseases caused by the pneumococcus bacterium comprising administering a vaccine that has the antigen of the present invention to a human or animal.

Therefore, in order to elucidate the present invention, experimental results and embodiments of the invention are presented below to demonstrate the inventive activity of the use of the antigens of the present invention.

### Implementations of the invention

Twenty epitopes from eight pneumococcal proteins that simultaneously stimulate the humoral and cellular arms were carefully selected. These are versatile epitopes derived from important pneumococcal proteins that are not allergenic or associated with autoimmunity in humans, totaling 453 amino acids (Table 2). Additionally, the ligands "KK" and "GPGPG" (SEQ ID NO: 24) were strategically incorporated into the antigen, giving it an average sum of 57 kDa.

**Table 2 - Selected epitopes from the eight pneumococcal proteins**

| SEQ ID NO: | Protein | Epitope | Overlap |
|---|---|---|---|
| 1 | NanA | TPDGKATDYRVVVDPVKPAY | B/MHC II/ MHCI |
| 2 | | LENATVHMEFKPDAKAPAFYNL FS | B/MHC II/ MHCI |
| 3 | | MAVYNNTATLEGRGSDGKQF | B/MHC II |
| 4 | PcsB | MSEIVSANNKMLEQQKADKK | B/MHC II/ MHCI |
| 5 | | DNKISNLTAQQQEAQKQVDQIQE Q | B/MHC II |
| 6 | PhtD | AGKYTTEDGYIFDPRDITSDEGD AY | B/MHC II/ MHCI |
| 7 | | KVGDGYVFEENGVPRYIPAK | B/MHC II |
| 8 | | GKYYVYLKDAAHADNIRTKE | B/MHC II |
| 9 | Ply | VNFKQIYYTVSVDAVKNPGDVF QDTVTVEDLKQRGI | B/MHC II/ MHCI |
| 10 | | QVYLKLETTSKSDEVEAAFE | B/MHC II |
| 11 | PncO | GLDLLLPSILFALPHFSSLPSLLDI FI | B/MHC II/ MHCI |
| 12 | | LIIQNYWQFSSQIGNFVWIQ | B/MHC II/ MHCI |
| 13 | | YSGTNFAELGIYITLFFLTP | B/MHCI |
| 14 | StkP | EKVEEGRIIRTDPGAGTGRK | B/MHC II |
| 15 | | VDLNKTRVKISIYKPKTTSATP | B/MHC II |
| 16 | | VTTAPAGSVEGMVVEQSPRA | B/MHC II/ MHCI |
| 17 | PspA-F1 | AAQRKYDEDQKKTEEKAKET | B/MHC II |
| 18 | | ARRSEEEYNRLPQQQLPKAE | B/MHC II |
| 19 | PspA-F2 | KKAYEEAKKKAEDAQKKYDEG QKKTEEKARK | B/MHC II |
| 20 | | EVQNAYVKYQRVQRNSRLNEKE R | B/MHC II/ MHCI |

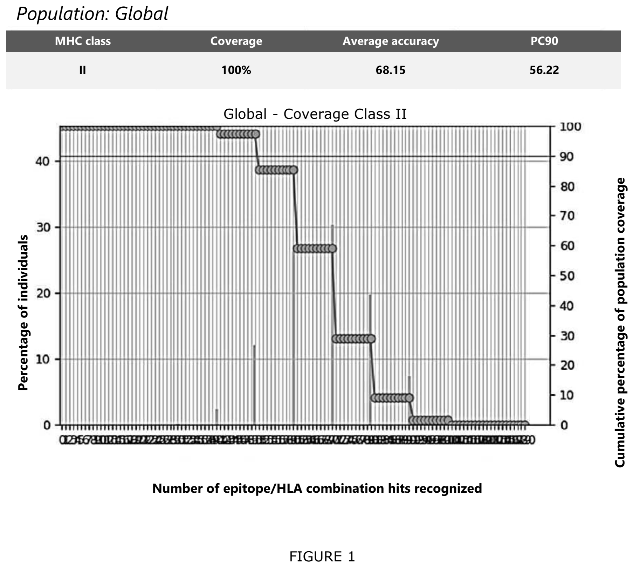

These epitopes were analyzed for coverage in the global population, showing 100% coverage (Figure 1).

Thus, these epitopes were arranged in different ways and 3 sequences yielded the best results: SEQ ID NO: 21, SEQ ID NO: 22 and SEQ ID NO: 23.

### Experimental Results

The aforementioned sequences were again analyzed for immunogenicity, allergenicity, and autoimmunity. Finally, the physicochemical properties, such as hydrophobicity and isoelectric point (pI), were verified.

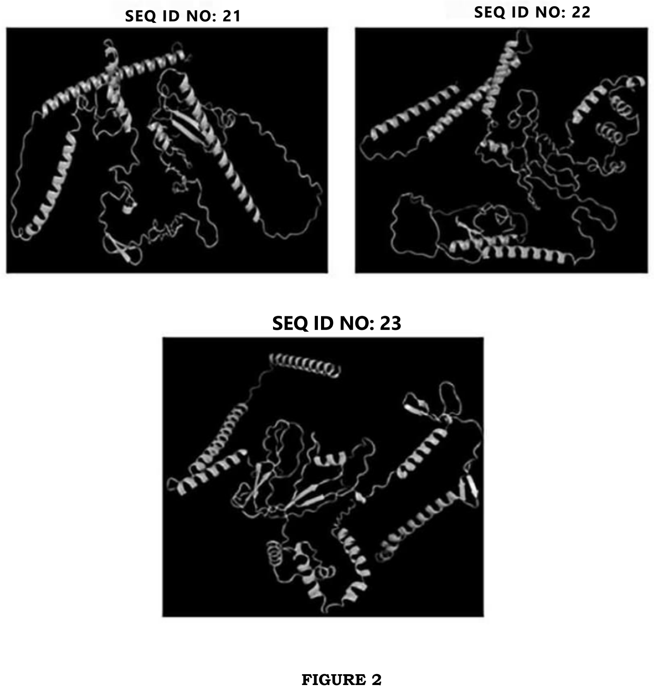

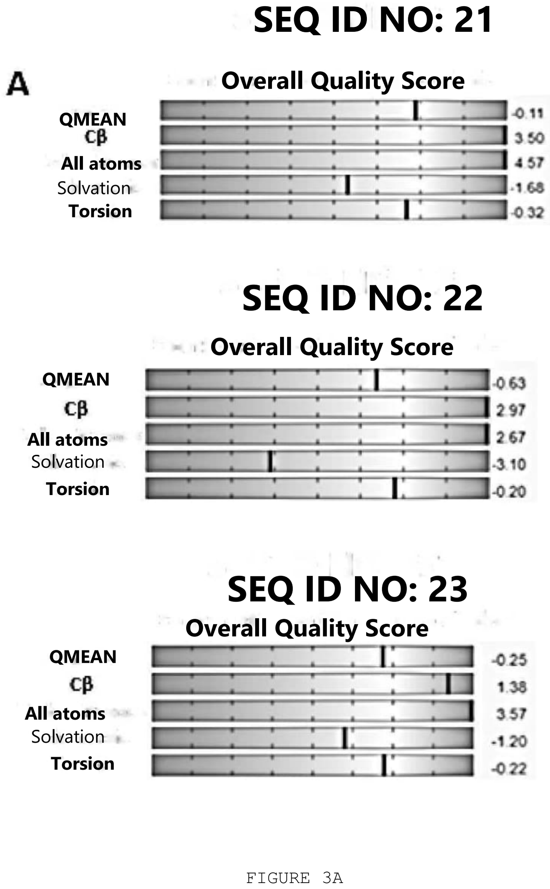

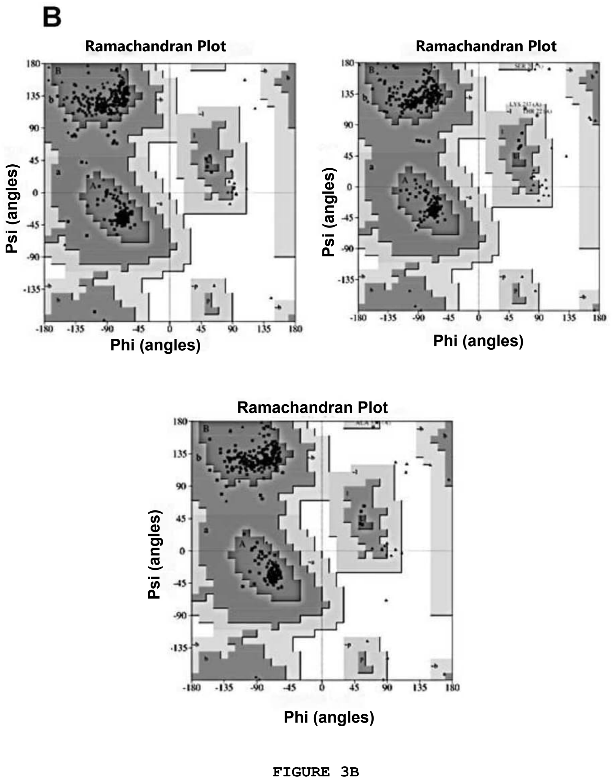

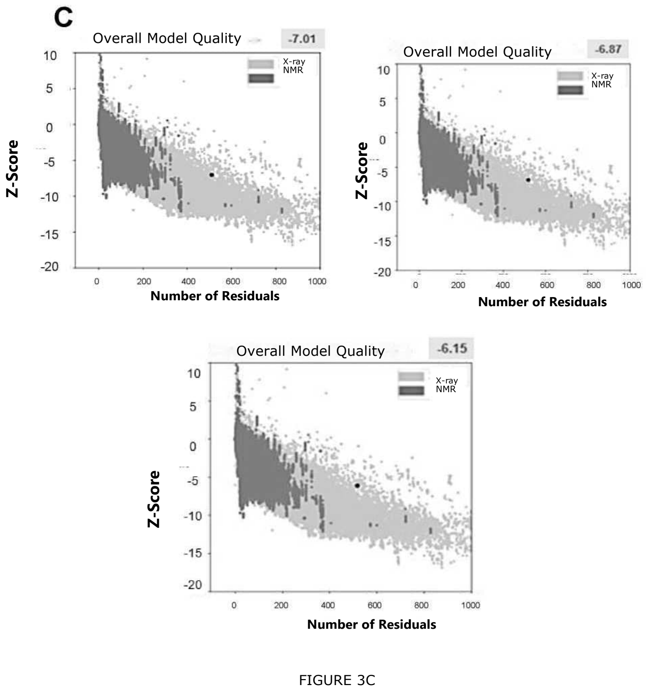

These sequences were used to generate the 3D models, using AlphaFold2 (Figure 2). Furthermore, the models were analyzed using bioinformatics tools regarding the stability of the 3 preferred sequences (Figure 3 AC), in order to increase the chances that the recombinantly produced antigens will have sufficient stability to be stored and distributed even to isolated areas of the world.

AlphaFold2 is an artificial intelligence program developed to predict the three-dimensional structure of proteins. Among its various applications, AlphaFold2 has been widely used in the resolution of vaccine candidates. However, predicting the three-dimensional structures of proteins can be difficult and often requires the use of other programs to validate the predicted structures, seeking a greater chance of identifying the best vaccine candidate. Most of protein structure validation programs take into account high-quality structures, resolved using X-ray crystallography and nuclear magnetic resonance. Some of these programs, Prosa-Web, PROCHECK, and QMEAN, were used to verify the quality of the structures predicted by AlphaFold2 and to identify which structures are the most reliable.

In the validation of the solved structures using QMEAN as an overall quality score, SEQ ID NO: 21 was the best, with -0.11, followed by SEQ ID NO: 23 (-0.25) and SEQ ID NO: 22 (-0.63) (Figure 3A). Stereochemical analysis, using Ramachandran plots from Procheck-SAVES, showed that SEQ ID NO: 21 was the only one without residues in energetically unfavorable regions, with 96.3% of amino acids in energetically favorable regions, while 3.7% were in additionally favorable regions. The sequence SEQ ID NO: 22 totaled 3 (0.7%) residues in generously allowed and non-allowed regions and 99.3% of residues in allowed or additionally allowed regions. Finally, SEQ ID NO: 23 had only 1 (0.2%) residue in generously allowed regions and 99.8% of the residues in energetically favorable regions (Figure 3B). The Prosa-Web data indicate that the 3D models of the sequences SEQ ID NO: 21, SEQ ID NO: 22, and SEQ ID NO: 23 had Z-score values (-7.01, -6.87, and -6.15) within the expected range for structures of the same size (Figure 3C). The validation data obtained by the QMEAN, Procheck-SAVES, and Prosa-Web programs for the three sequences suggest that they would have adequate folding and would configure biologically stable structures.

Thus, through the use of immunobioinformatics tools, it was possible to demonstrate:
(i) The exploration of the organization of the selected epitopes in the three sequences (SEQ ID NOs: 21 to 23), introducing the concept of blocks and, seeking increased stability, adapting the sequences to contain epitopes with valine at their beginning;
(ii) Novel polypeptide chains, not found in nature, as well as the fact that multi-epitope proteins can present expression problems, therefore, as proposed by the present invention, in addition to optimizing the codons of the gene sequences for expression in *E. coli,* a synthetic biology program, *TIsigner,* was also used to optimize the gene at the messenger RNA level, helping to reduce its energy, which facilitates opening by the ribosome, with a consequent increase in the synthesis of the protein of interest;
(iii) Choice of epitopes considering purification and yield, in which the highest isoelectric points (pI) were selected. Thus, three high pI (>9) sequences were generated that can be purified by ion-exchange chromatography, dispensing with the His-tag or any other affinity tail.

Therefore, the results presented here showed that the three antigen embodiments of the present invention, multi-epitope vaccine candidates, were rationally designed to act as serotype-independent vaccines, against the 100 pneumococcal serotypes existing in the world, stable, safe and with broad coverage in global populations, constituting a cheaper alternative to existing polysaccharide-based conjugate vaccines on the market.

In short, the invention relates to the following aspects, as defined in the following numbered items:
1. Multi-epitope antigen comprising 20 epitope blocks of 20 to 36 amino acids from proteins selected from the group consisting of NanA, PcsB, PhtD, Ply, PncO, StkP, PspA-F1 and PspA-F2.
2. Multi-epitope antigen, according to item 1, wherein said epitopes are selected from the group consisting of MHC I, MHC II and B epitopes.
3. Multi-epitope antigen, according to item 1 or 2, wherein it preferably comprises 3 blocks of the NanA protein, 2 blocks of the PcsB protein, 3 blocks of the PhtD protein, 2 blocks of the Ply protein, 3 blocks of the PncO protein, 3 blocks of the StkP protein, 2 blocks of the PspA-F1 protein, and 2 blocks of the PspA-F2 protein.
4. Multi-epitope antigen, according to item 3, wherein said epitope blocks are selected from the group consisting of amino acid sequences as established by SEQ ID NOs: 1 to 20, wherein:
   (i) SEQ ID NOs: 1 to 3 refer to the NanA protein blocks;
   (ii) SEQ ID NOs: 4 and 5 refer to the PcsB protein blocks;
   (iii) SEQ ID NOs: 6 to 8 refer to the PhtD protein blocks;
   (iv) SEQ ID NOs: 9 and 10 refer to the Ply protein blocks;
   (v) SEQ IDs NOs: 11 to 13 refer to the PncO protein blocks;
   (vi) SEQ IDs NOs: 14 to 16 refer to the StkP protein blocks;
   (vii) SEQ IDs NOs: 17 and 18 refer to the PspA-F1 protein blocks
   (viii) and SEQ IDs NOs: 19 and 20 refer to the PspA-F2 protein blocks.
5. Multi-epitope antigen, according to any of items 1 to 4, which further includes the ligand as defined by SEQ ID NO: 24 and the "KK" ligand, wherein the SEQ ID NO: 24 ligand is used to separate the blocks, and the "KK" ligand is used to separate each epitope within the blocks.
6. Multi-epitope antigen, according to any of items 1 to 5, which further comprises a methionine (M) at the beginning of the amino acid sequence thereof formed by the 20 epitopes, wherein M may or may not be part of said epitopes.
7. Multi-epitope antigen, according to any of items 1 to 6, comprising a random order of said 20 epitopes linked by the SEQ ID NO: 24 and "KK" ligands, wherein the epitopes were preferably ordered by block logic.
8. Multi-epitope antigen, according to any of items 1 to 6, comprising an order of the aforementioned 20 epitopes linked by SEQ ID NO: 24 and "KK" ligands, organized by the GRAVY index of each epitope, where preferably the epitopes are ordered in such a way as to keep the most hydrophobic ones in the center of the molecule and the most hydrophilic ones at the ends.
9. Multi-epitope antigen, according to any of items 1 to 8, comprising the following epitope order: SEQ ID NO: 16- SEQ ID NO: 15- SEQ ID NO: 14- SEQ ID NO: 5- SEQ ID NO: 4- SEQ ID NO: 9- SEQ ID NO: 10- SEQ ID NO: 2- SEQ ID NO: 3- SEQ ID NO: 1- SEQ ID NO: 8- SEQ ID NO: 7- SEQ ID NO: 6- SEQ ID NO: 12-13- SEQ ID NO: 11- SEQ ID NO: 17- SEQ ID NO: 18- SEQ ID NO: 19 and - SEQ ID NO: 20.
10. Multi-epitope antigen, according to any of items 1 to 8, comprising the following epitope order: SEQ ID NO:15- SEQ ID NO:20- SEQ ID NO:14- SEQ ID NO:6- SEQ ID NO:3- SEQ ID NO:17- SEQ ID NO:7- SEQ ID NO:10- SEQ ID NO:9-SEQ ID NO:13-11- SEQ ID NO:12- SEQ ID NO:16- SEQ ID NO:2- SEQ ID NO:1- SEQ ID NO:8- SEQ ID NO:4- SEQ ID NO:5- SEQ ID NO:18 and - SEQ ID NO:19.
11. Multi-epitope antigen, according to any of items 1 to 8, comprising the following epitope order: SEQ ID NO: 3- SEQ ID NO:20- SEQ ID NO:14- SEQ ID NO:6- SEQ ID NO:17- SEQ ID NO:15- SEQ ID NO:7- SEQ ID NO:10- SEQ ID NO:9-SEQ ID NO:13- SEQ ID NO:11- SEQ ID NO:12- SEQ ID NO:16- SEQ ID NO:2- SEQ ID NO:1- SEQ ID NO:8- SEQ ID NO:4- SEQ ID NO:5- SEQ ID NO:18- and SEQ ID NO:19.
12. Multi-epitope antigen, according to any of items 1 to 11, consisting of the amino acid sequence as established by SEQ ID NOS: 21 to 23.
13. Immunogenic composition comprising the multi-epitope antigen as defined in items 1 to 12, and a pharmaceutically acceptable vehicle and/or adjuvant.
14. Immunogenic composition, according to item 13, preferably a pneumococcal vaccine.
15. Immunogenic composition, according to item 13 or 14, administered parenterally, which includes subcutaneous, intravenous, epidural, intramuscular, delivery pumps, or infusion.
16. Immunogenic composition, according to item 13 or 14, administered nasally, directed or not to the lungs.
17. Pneumococcal diagnostic kit comprising the multi-epitope antigen as defined in items 1 to 12 or a biologically active fragment thereof bound to a detectable fraction; at least one antibody generated by the antigen as defined in items 1 to 12; and instructions for use.
18. Kit, according to item 17, preferably an immunochromatographic kit or an ELISA.
19. Use of the multi-epitope antigen as defined in items 1 to 12 for the preparation of a vaccine to prevent diseases caused by pneumococcal bacteria.
20. Use of the multi-epitope antigen as defined in items 1 to 12 for the production of monoclonal and polyclonal antibodies.
*21. In vitro* diagnostic method for pneumococcus comprising applying the multi-epitope antigen as defined in items 1 to 12 or a biologically active fragment thereof bound to a detectable fraction, in contact with samples of selected body fluids from blood, mucus expelled from the lower airways and urine.

Thus, the embodiments presented in this invention do not limit the totality of possibilities, and it will be understood that various omissions, substitutions, and alterations may be made by a person skilled in the art, without departing from the scope of the present invention.

It is expressly provided that all combinations of elements that perform the same function substantially in the same way to achieve the same results are within the scope of the invention. Substitutions of elements from one described embodiment to another are also fully intended and contemplated.

Those skilled in the art will appreciate the knowledge presented herein and may reproduce the invention in the embodiments presented and in other variants, covered within the scope of the claims.

## Claims

1. Multi-epitope antigen **characterized in that** it comprises 20 epitope blocks of 20 to 36 amino acids from proteins selected from the group consisting of NanA, PcsB, PhtD, Ply, PncO, StkP, PspA-F1 and PspA-F2.

2. Multi-epitope antigen, according to claim 1, **characterized in that** said epitopes are selected from the group consisting of MHC I, MHC II and B epitopes.

3. Multi-epitope antigen, according to claim 1 or 2, **characterized in that** it preferably comprises 3 blocks of the NanA protein, 2 blocks of the PcsB protein, 3 blocks of the PhtD protein, 2 blocks of the Ply protein, 3 blocks of the PncO protein, 3 blocks of the StkP protein, 2 blocks of the PspA-F1 protein, and 2 blocks of the PspA-F2 protein.

4. Multi-epitope antigen, according to claim 3, **characterized in that** said epitope blocks are selected from the group consisting of amino acid sequences as established by SEQ ID NOs: 1 to 20, wherein:
(i) SEQ ID NOs: 1 to 3 refer to the NanA protein blocks;
(ii) SEQ ID NOs: 4 and 5 refer to the PcsB protein blocks;
(iii) SEQ ID NOs: 6 to 8 refer to the PhtD protein blocks;
(iv) SEQ ID NOs: 9 and 10 refer to the Ply protein blocks;
(v) SEQ IDs NOs: 11 to 13 refer to the PncO protein blocks;
(vi) SEQ IDs NOs: 14 to 16 refer to the StkP protein blocks;
(vii) SEQ IDs NOs: 17 and 18 refer to the PspA-F1 protein blocks
(viii) and SEQ IDs NOs: 19 and 20 refer to the PspA-F2 protein blocks.

5. Multi-epitope antigen, according to any one of claims 1 to 4, **characterized in that** it further comprises the ligand as set forth by SEQ ID NO: 24, and the "KK" ligand, wherein the SEQ ID NO: 24 ligand is used to separate the blocks, and the "KK" ligand is used to separate each epitope within the blocks.

6. Multi-epitope antigen, according to any one of claims 1 to 5, **characterized in that** it further comprises a methionine (M) at the beginning of the amino acid sequence thereof formed by the 20 epitopes, wherein M may or may not be part of said epitopes.

7. Multi-epitope antigen, according to any one of claims 1 to 6, **characterized in that** it comprises a random order of said 20 epitopes linked by the SEQ ID NO: 24 and "KK" ligands, wherein the epitopes have preferably been ordered by block logic.

8. Multi-epitope antigen, according to any one of claims 1 to 6, **characterized in that** it comprises an order of said 20 epitopes linked by the SEQ ID NO: 24 and "KK" ligands arranged by the index of GRAVY analysis of each epitope, in which the epitopes are preferably ordered in such a way as to keep the most hydrophobic ones in the center of the molecule and the most hydrophilic ones at the ends.

9. Multi-epitope antigen, according to any one of claims 1 to 8, **characterized in that** it comprises the following epitope order: SEQ ID NO: 16- SEQ ID NO: 15- SEQ ID NO: 14-SEQ ID NO: 5- SEQ ID NO: 4- SEQ ID NO: 9- SEQ ID NO: 10- SEQ ID NO: 2- SEQ ID NO: 3- SEQ ID NO: 1- SEQ ID NO: 8- SEQ ID NO: 7- SEQ ID NO: 6- SEQ ID NO: 12-13- SEQ ID NO: 11- SEQ ID NO: 17- SEQ ID NO: 18- SEQ ID NO: 19 and - SEQ ID NO: 20.

10. Multi-epitope antigen, according to any one of claims 1 to 8, **characterized in that** it comprises the following epitope order: SEQ ID NO: 15- SEQ ID NO: 20- SEQ ID NO: 14-SEQ ID NO: 6- SEQ ID NO: 3- SEQ ID NO: 17- SEQ ID NO: 7- SEQ ID NO: 10- SEQ ID NO: 9- SEQ ID NO: 13- 11- SEQ ID NO: 12- SEQ ID NO: 16- SEQ ID NO: 2- SEQ ID NO: 1- SEQ ID NO: 8- SEQ ID NO: 4- SEQ ID NO: 5 - SEQ ID NO: 18 and - SEQ ID NO: 19.

11. Multi-epitope antigen, according to any one of claims 1 to 8, **characterized by the fact that** it comprises the following epitope order: SEQ ID NO: 3- SEQ ID NO: 20- SEQ ID NO: 14- SEQ ID NO: 6- SEQ ID NO: 17- SEQ ID NO: 15- SEQ ID NO: 7- SEQ ID NO: 10- SEQ ID NO: 9- SEQ ID NO: 13- SEQ ID NO: 11- SEQ ID NO: 12- SEQ ID NO: 16-SEQ ID NO: 2- SEQ ID NO: 1- SEQ ID NO: 8- SEQ ID NO: 4- SEQ ID NO: 5- SEQ ID NO: 18- and SEQ ID NO: 19.

12. Multi-epitope antigen, according to any one of claims 1 to 11, **characterized in that** it consists of the amino acid sequence as set forth by SEQ ID NOS: 21 to 23.

13. Immunogenic composition **characterized in that** it comprises the multi-epitope antigen as defined by claims 1 to 12, and a pharmaceutically acceptable vehicle and/or adjuvant.

14. Immunogenic composition, according to claim 13, **characterized in that** it is preferably a pneumococcal vaccine.

15. Immunogenic composition, according to claim 13 or 14, **characterized in that** it is administered parenterally, which includes subcutaneous, intravenous, epidural, intramuscular, delivery pumps, or infusion.

16. Immunogenic composition, according to claim 13 or 14, **characterized in that** it is administered nasally, directed or not to the lungs.

17. Pneumococcal diagnostic kit **characterized in that** it comprises the multi-epitope antigen as defined in claims 1 to 12 or a biologically active fragment thereof bound to a detectable fraction; at least one antibody generated by antigen as defined in claims 1 to 12; and instructions for use.

18. Kit, according to claim 17, **characterized in that** it is preferably an immunochromatographic or ELISA kit.

19. Use of the antigen as defined in claims 1 to 12 **characterized in that it is** for the preparation of a vaccine to prevent diseases caused by pneumococcal bacteria.

20. Use of the multi-epitope antigen as defined in claims 1 to 12, **characterized by** being for the production of monoclonal and polyclonal antibodies.

21. *In vitro* diagnostic method for pneumococcus **characterized in that** it comprises applying the multi-epitope antigen as defined in claims 1 to 12, or a biologically active fragment thereof bound to a detectable fraction, in contact with samples of selected body fluids from blood, mucus expelled from the lower airways, and urine.
